# EUROPEAN PATENT SPECIFICATION

(11) **EP 3 807 260 B1**
(45) Date of publication and mention of the grant of the patent: **18.09.2024**
(21) Application number: 19755429.8
(22) Date of filing: 12.06.2019
(51) Int. Cl.: C07D 401/04

(54) **PURIFICATION OF NICOTINE**
REINIGUNG VON NIKOTIN
PURIFICATION DE NICOTINE

(30) Priority: 15.06.2018 US 201862685573 P
(43) Date of publication of application: 21.04.2021
(73) Proprietor: R. J. Reynolds Tobacco Company, Winston-Salem, North Carolina 27101 (US)
(72) Inventor: PARTAIN, Nicholas, Owensboro, Kentucky 42301 (US); MORTON, Joshua D., Evansville, Indiana 47712 (US); SIMPSON, Carrie A., Evansville, Indiana 47715 (US); DULL, Gary M., Lewisville, North Carolina 27023 (US); BRATCHER, Barry, Owensboro, Kentucky 42303 (US)
(74) Representative: Dehns
(86) International application number: PCT/IB2019/054927
(87) International publication number: WO 2019/239356

(56) References cited:
- WO-A1-2014/009749
- CN-A- 105 566 288

## Description

### FIELD OF THE DISCLOSURE

The present disclosure relates primarily to isolation and purification of active ingredients, as well as to compositions and products that contain such active ingredients.

### BACKGROUND

Central nervous system (CNS) conditions, diseases, or disorders can be drug induced; can be attributed to genetic predisposition, infection or trauma; or can be of unknown etiology. They comprise neuropsychiatric disorders, neurological diseases and mental illnesses; and include neurodegenerative diseases, behavioral disorders, cognitive disorders and cognitive affective disorders. The clinical manifestations of several CNS conditions, diseases or disorders have been attributed to CNS dysfunction (i.e., disorders resulting from inappropriate levels of neurotransmitter release, inappropriate properties of neurotransmitter receptors, and/or inappropriate interaction between neurotransmitters and neurotransmitter receptors).

Nicotinic compounds, such as nicotine, are capable of affecting nicotinic acetylcholinergic receptors (nAChRs). Subtypes of nAChRs exist in both the CNS and the peripheral nervous system (PNS), but the distribution of subtypes is heterogeneous. For instance, certain subtypes are predominant in vertebrate brain, others predominate at the autonomic ganglia, and others predominate at the neuromuscular junction. Activation of nAChRs by nicotinic compounds results in neurotransmitter release. See, for example, Dwoskin et al., Exp. Opin. Ther. Patents, 10: 1561-1581 (2000); Schmitt et al., Annual Reports in Med. Chem., 35: 41-51 (2000); Huang et al., J. Am. Chem. Soc., 127: 14401-14414 (2006); Americ et al., Biochem. Pharmacol., 74: 1092-1101 (2007) and Millar, Biochem. Pharmacol., 78: 766-776 (2009).

It has been suggested that administration of nicotine, and other nicotinic compounds, can result in various pharmacological effects. See, for example, US Pat. Nos. 5,583,140 to Bencherifet al.; 5,723,477 to McDonald et al.; 7,001,900 to Jacobsen et al.; 7,135,484 to Dart et al. and 7,214,686 to Bencherifet al.; and US Pat. Pub. Nos. 2010/0004451 to Ahmad et al. and 2011/0274628 to Borschke. As a result, it has been suggested that nicotine, and other nicotinic compounds, can exhibit utility as active ingredients in the treatment of a wide variety of conditions, diseases, and disorders, including those that affect the CNS. Additionally, administration of nicotine and nicotinic compounds has been proposed for treatment of certain other conditions, diseases, and disorders. See, for example, US Pat. Nos. 5,604,231 to Smith et al.; 5,811,442 to Bencherifet al.; 6,238,689 to Rhodes et al. and 6,489,349 to Bencherif et al. Furthermore, administration of nicotine has been employed in an effort to help cigarette smokers quit smoking (i.e., as a smoking cessation aid). For example, nicotine has been an active ingredient of various types of so-called "nicotine replacement therapy" or "NRT" products. See, for example, the background art set forth in US Pat. Pub. No. 2011/0268809 Brinkley et al.

It has been proposed to administer nicotine using a transdermal patch. Representative types of nicotine-containing transdermal patch products have been marketed under the tradenames "Habitrol," "Nicoderm," "Nicorette," "Nicorette CQ," "Nicotinell" and "ProStep." See also, for example, US Pat. Nos. 4,597,961 to Etscom; 5,298,257 to Bannon et al.; 5,603,947 to Wong et al.; 5,834,011 to Rose et al.; 6,165,497 to Osborne et al. and 6,676,959 to Anderson et al. It also has been suggested that transdermal administration of nicotine can be accompanied by ingestion of other types of nicotine-containing products. See, for example, US Pat. No. 5,593,684 to Baker et al.; US Pat. Pub. No. 2009/0004249 to Gonda and Fagerstrom, Health Values, 18:15 (1994).

One particularly popular way to provide for oral administration of nicotine has been through the use of nicotine-containing gum or other type of similarly chewable product. Gum forms of product generally include a gum base (e.g., typically the types of pharmaceutically acceptable gum bases available from sources such as Gum Base Co. S.p.a., Wm. J. Wrigley Jr. Company or Gumlink A/S). See, for example, the types of nicotine-containing gums, gum formulations, gum formats and configurations, gum characteristics and techniques for formulating or manufacturing gums set forth in US Pat. Nos. 3,845,217 to Ferno et al.; 3,877,468 to Lichtneckert et al.; 3,901,248 to Lichtneckert et al.; 4,317,837 to Kehoe et al.; 4,802,498 to Ogren; 5,154,927 to Song et al.; 6,322,806 to Ream et al.; 6,344,222 to Cherukuri et al.; 6,355,265 to Ream et al.; 6,358,060 to Pinney et al.; 6,773,716 to Ream et al.; 6,893,654 to Pinney et al.; 7,101,579 Athanikar et al.; 7,163,705 to Johnson et al. and 7,208,186 to Norman et al.; US Pat. Pub. Nos. 2004/0191322 to Hansson; 2004/0194793 to Lindell et al.; 2006/0099300 to Andersen et al.; 2006/0121156 to Andersen et al.; 2006/0165842 to Andersen et al.; 2006/0204451 to Salini; 2006/0246174 to Andersen et al.; 2006/0275344 to Mody et al.; 2007/0014887 to Cherukuri et al.; 2007/0269386 to Steen et al.; 2009/0092573 to Andersen and 2010/0061940 to Axelsson et al. Representative nicotine-containing gum products have been marketed under the tradenames "Nicorette," "Nicotinell" and "Zonnic."

Another way that has been employed to provide oral administration of nicotine has been through the use of nicotine-containing lozenge or tablet types of products. Nicotine-containing lozenge, mini lozenge, tablet, and microtab types of products have been marketed under the tradenames "Commit," "Nicorette," "Nicotinell" and "NiQuitin." See also, for example, US Pat. Nos. 5,110,605 to Acharya; 5,733,574 to Dam; 6,280,761 to Santus; 6,676,959 to Andersson et al. and 6,248,760 to Wilhelmsen; US Pat. Pub. Nos. 2001/0016593 to Wilhelmsen and 2010/0004294 to Axelsson et al.

A further method that has been employed to provide oral administration of nicotine has been through the use of nicotine-containing pouches or sachet types of products. See, for example, the types of pouch materials and nicotine-containing formulations set forth in US Pat. No. 4,907,605 to Ray et al. and US Pat. Pub. No. 2009/0293895 to Axelsson et al. See also, for example, the types of pouch materials and pouch manufacturing techniques (e.g., pouch filling and sealing techniques) set forth in US Pat. Pub. No. 2010/0018539 to Brinkley et al. Representative nicotine-containing pouch-type products have been marketed under the tradename "Zonnic."

Attempts have been made to incorporate nicotine into beverages (e.g., water, juices, coffee and so-called fortified beverages). See, for example, US Pat. Nos. 6,211,194 to Westman et al.; 6,268,386 to Thompson; 6,749,882 to Fortune, Jr.; 7,115,297 to Stillman and 7,435,749 to Knight. Additionally, attempts have been made to market nicotine-containing beverages, such as certain types of beverages have been introduced commercially under the tradenames "Nic Lite," "Nico Water," "Nic Med," and Nico Shot."

Nicotine also has been administered in inhalable form, such as in the form of nasal or oral sprays. Typically, sprays are applied within the nose or mouth for absorption through nasal or oral mucosa. Various exemplary ways to administer nicotine in the form of a nasal spray are set forth in US Pat. Nos. 4,579,858 to Ferno et al.; 5,656,255 to Jones and 6,596,740 to Jones. Various exemplary ways to administer nicotine in the form of an oral spray, such as for buccal administration, are set forth in US Pat. Nos. 6,024,097 to Von Wielligh; US Pat. Pub. Nos. 2003/0159702 to Lindell et al.; 2007/0163610 to Lindell et al. and 2009/0023819 to Axelsson; EP 1458388 to Lindell et al. and PCT WO 2008/037470 to Axelsson et al. Various other types of inhalable formulations, and various vapor delivery devices and systems, are set forth in US Pat. Nos. 4,284,809 to Ray; 4,800,903 to Ray et al.; 5,167,242 to Turner et al.; 6,098,632 to Turner et al.; 6,234,169 to Bulbrook et al. and 6,874,507 to Farr; US Pat. Pub. Nos. 2004/0034068 to Warchol et al; 2006/0018840 to Lechuga-Ballesteros; 2008/0302375 to Andersson et al. and 2009/0005423 to Gonda. Representative nicotine-containing spray-type and inhalation types of products have been marketed under the tradenames "Favor," "Nicotrol NS," "Quit" and "Zonnic."

There also have been proposed numerous smoking products, flavor generators and medicinal inhalers that utilize electrical energy to vaporize or heat volatile materials (e.g., formulations that incorporate components such as tobacco-derived nicotine, glycerin, propylene glycol, organic acids and flavors), or otherwise attempt to provide the sensations of cigarette, cigar or pipe smoking without burning tobacco to a significant degree. See, for example, the various alternative smoking articles, aerosol delivery devices and heat generating sources set forth in the background art described in U.S. Pat. Nos. 7,726,320 to Robinson et al. and 8,881,737 to Collett et al. See also, for example, the various types of smoking articles, aerosol delivery devices and electrically-powered heat generating sources referenced by brand name and commercial source in U.S. Pat. Pub. No. 2015/0216232 to Bless et al. Additionally, various types of electrically powered aerosol and vapor delivery devices also have been proposed in U.S. Pat. Pub. Nos. 2014/0096781 to Sears et al. and 2014/0283859 to Minskoff et al., as well as U.S. Pat. App. Ser. Nos. 14/282,768 to Sears et al., filed May 20, 2014; 14/286,552 to Brinkley et al., filed May 23, 2014; 14/327,776 to Ampolini et al., filed July 10, 2014; and 14/465,167 to Worm et al., filed August 21, 2014.

Various other ways to provide a source of nicotine, or to administer nicotine, have been proposed. For example, it has been suggested that nicotine can be incorporated into orally dissolving films (e.g., US Pat. Nos. 6,709,671 to Zerbe et al.; 7,025,983 to Leung et al. and 7,491,406 to Leung et al.; and US Pat. Pub. Nos. 2006/0198873 to Chan et al.; 2006/0204559 to Bess et al. and 2010/0256197 to Lockwood et al.); oral osmotic devices (e.g., US Pat. No. 5,147,654 to Place et al.); gum pads (e.g., US Pat. No. 6,319,510 to Yates); oral patches (e.g., US Pat. Pub. No. 2006/0240087 to Houze et al.); lip balm (e.g., US Pat. No. 7,105,173 to Rolling); dentifrice compositions and toothpicks (e.g., US Pat. Nos. 5,176,899 to Montgomery; 5,035,252 to Mondre; 5,560,379 to Pieczenik; and US Pat. Pub. Nos. 2004/0025900 to Sampson; 2005/0058609 to Nazeri and 2006/0162732 to Winn); and other forms (e.g., US Pat. Nos. 5,048,544 to Mascarelli; 6,082,368 to Brown; 6,319,510 to Yates and 6,949,264 to McGrew et al.; and US Pat. Pub. Nos. 2005/0008735 to Pearce).

CN 105566288 discloses an industrialized process for preparing high purity nicotine from waste tobacco.

WO 2014/009749 discloses a process for deriving two or more components or products from tobacco biomass stock, wherein at least one component or product is extracted from a liquid phase, and at least one component or product is produced from a solid phase. The component or product extracted from a liquid phase may be nicotine.

As such, obtaining nicotine is of importance to a number of applications, and it would be desirable to provide further methods for obtaining and purifying nicotine for use in such compositions and devices.

### BRIEF SUMMARY

The present disclosure provides a method for purifying nicotine from fresh (green) tobacco.

The method for providing a nicotine isolate comprises: a) receiving a solution comprising nicotine derived from green tobacco biomass of a plant of the *Nicotiana* species which has not been cured; b) converting the nicotine to nicotine sulfate, giving a nicotine sulfate-containing solution; c) concentrating the nicotine sulfate-containing solution to give a nicotine sulfate concentrate; d) adjusting the pH of the nicotine sulfate concentrate to a pH of about 9.5 or greater to convert the nicotine sulfate to nicotine in free base form, providing a basic concentrate; e) extracting the basic concentrate with an organic solvent to partition the nicotine in free base form into the organic solvent, providing a nicotine-containing organic solution; and f) distilling the nicotine-containing organic solution to afford a nicotine isolate, wherein the nicotine isolate comprises about 90% or more nicotine by weight. Advantageously, the nicotine isolate can, in some embodiments, comprise even higher amounts of nicotine by weight, e.g., about 92% or more, about 95% or more, about 97% or more, about 98% or more, or about 99% or more nicotine by weight.

The solution in step a) referenced above is a byproduct of a method to provide protein from tobacco. For example, the solution may be a distillate of a method to provide protein from tobacco.

In some embodiments, the converting in step b) referenced above comprises treating the solution comprising nicotine with sulfuric acid. In some embodiments, the nicotine sulfate-containing solution has a pH of about 2 to about 6. In some embodiments, the nicotine sulfate-containing solution comprises about 90% or more nicotine sulfate by dry weight. The nicotine sulfate-containing solution, in various embodiments, comprises about 3% or less myosmine by dry weight, and/or about 2% or less nicotine N-oxide by dry weight.

In certain embodiments, the concentrating in step c) referenced above comprises subjecting the nicotine sulfate-containing solution to vacuum evaporation or distillation. In some embodiments, the concentrating in step c) comprises subjecting the nicotine sulfate-containing solution to counter-current extraction. The specific parameters of the concentrating method can vary. For example, where the concentrating comprises vacuum evaporation, certain non-limiting conditions include evaporation at a temperature of about 40°C to about 50°C and a pressure of about -25 in Hg to -27 in Hg (16.7 kPa to 9.90 kPa).

In some embodiments, the adjusting in step d) referenced above comprises adding a sodium hydroxide solution to the nicotine sulfate concentrate. The pH obtained via this adjusting step can vary. For example, in certain embodiments, the adjusting comprises adjusting to a pH of about 12 or greater. In some embodiments, the adjusting comprises adjusting to a pH of about 13.

In certain embodiments, the method further comprises removing solids from the basic concentrate prior to step e) referenced above. For example, removing solids may comprise filtering the solids from the basic concentrate. In certain embodiments, there is no intervening processing step between steps d) and e) referenced above. For example, there may be no filtration, no distillation, no extraction, etc. between these referenced method steps.

In some specific embodiments, the organic solvent in step e) referenced above comprises cyclohexane. The nicotine-containing organic solution in step e) may, in some embodiments, comprise at least about 40% nicotine by weight.

In some embodiments, the distilling of step f) referenced above comprises a first stage to remove organic solvent and a second stage to distill and collect the nicotine isolate. In some such embodiments, the first stage is conducted at elevated temperature and atmospheric pressure and wherein the second stage is conducted under vacuum.

These and other features, aspects, and advantages of the present disclosure will be apparent from a reading of the following detailed description together with the accompanying drawings, which are briefly described below. The present disclosure includes any combination of two, three, four, or more features or elements set forth in this disclosure or recited in any one or more of the claims, regardless of whether such features or elements are expressly combined or otherwise recited in a specific embodiment description or claim herein. This disclosure is intended to be read holistically such that any separable features or elements of the disclosure, in any of its aspects and embodiments, should be viewed as intended to be combinable, unless the context of the disclosure clearly dictates otherwise.

### BRIEF DESCRIPTION OF THE DRAWINGS

In order to provide an understanding of embodiments of the disclosure, reference is made to the appended drawings, which are not necessarily drawn to scale, and in which reference numerals refer to components of exemplary embodiments. The drawings are exemplary only, and should not be construed as limiting the scope of the disclosure.
FIG. 1 is a flowchart of method steps associated with one embodiment of the present disclosure;
FIG. 2 is a flowchart of method steps associated with another embodiment of the present disclosure; and
FIG. 3 is a sectional view through a control body of an electronic smoking article according to one embodiment disclosed herein.

### DETAILED DESCRIPTION OF THE PREFERRED EMBODIMENTS

The present invention now will be described more fully hereinafter. This invention may, however, be embodied in many different forms and should not be construed as limited to the embodiments set forth herein; rather, these embodiments are provided so that this disclosure will be thorough and complete, and will fully convey the scope of the invention to those skilled in the art. As used in this specification and the claims, the singular forms "a," "an," and "the" include plural referents unless the context clearly dictates otherwise. Reference to "dry weight percent" or "dry weight basis" refers to weight on the basis of dry ingredients (i.e., all ingredients except water).

The present disclosure provides methods for obtaining and/or purifying nicotine-containing materials derived from plant biomass. Nicotine (3-(1-methylpyrrolidin-2-yl)pyridine) is a compound produced by certain plants, e.g., plants of the *Nicotiana* species. Nicotine can have the enantiomeric form S(-)-nicotine, R(+)-nicotine, or a mixture of S(-)-nicotine and R(+)-nicotine. Most preferably, nicotine provided and/or purified according to the disclosed methods is in the form of S(-)-nicotine (e.g., in a form that is virtually all S(-)-nicotine) or a racemic mixture composed primarily or predominantly of S(-)-nicotine (e.g., a mixture composed of about 95 weight parts S(-)-nicotine and about 5 weight parts R(+)-nicotine). It is noted that tobacco-derived nicotine is typically about 99.3% S enantiomer with -0.7% R enantiomer; while the nicotine provided according to the present disclosure may be consistent with (or close to) this ratio, it is not limited thereto.

The term "biomass" and related terms such as "biomatter" and "plant source" are understood to refer to any portion of a harvested plant that may be processed to extract, separate, or isolate components of interest therefrom. The processing may be carried out in relation to various plants or portions thereof, such as seeds, flowers, stalks, stems, roots, tubers, leaves, or any further portions of the plant.

The present disclosure more specifically provides methods for obtaining and/or purifying nicotine from plant biomass-derived, nicotine-containing materials from a plant of the *Nicotiana* species. Certain such methods, described herein, provide nicotine in a purified form, e.g., suitable for use in aerosol delivery devices and pharmaceutical products. According to the disclosed methods, nicotine is obtained and purified from fresh plant biomass. By "fresh" plant biomass is meant biomass that has not been cured (e.g., via traditional tobacco curing processes). Such fresh plant biomass can also be referred to as "green" biomass.

Traditional processes for the extraction and purification of nicotine involve deriving the nicotine from cured tobacco material. According to the present disclosure, the processing of fresh/green tobacco material (rather than cured tobacco material) to obtain nicotine simplifies the process of providing nicotine of the desired purity. Typically, fresh tobacco material comprises less impurities such as nitrosamines, than corresponding cured material. As such, by providing a method by which nicotine can be derived from fresh/green tobacco, different and fewer processing steps can be required. For example, the process can involve fewer steps and/or can replace certain reagents/processes traditionally required with milder reagents/simpler processes. In some embodiments, chromatography is not required (although the disclosed methods are not to be read to necessarily exclude chromatographic steps). In some embodiments, the process can result in the generation of less hazardous waste (requiring disposal or storage) than typical nicotine extraction/purification processes and, in some embodiments, the process can result in minimized overall cost associated with obtaining nicotine of the desired purity as compared with typical nicotine extraction/purification processes.

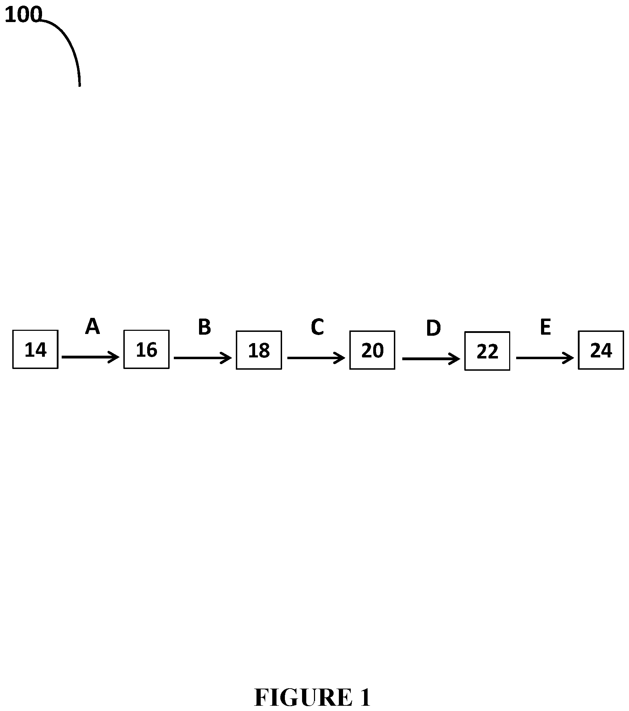

The process of the present disclosure generally can be outlined as depicted in FIG. 1. In FIG. 1, process **100** for the isolation and purification of nicotine begins with obtaining a nicotine-containing material **14.** Nicotine-containing material **14,** as referenced above, is a material derived from a plant of the *Nicotiana* species, and has not been subjected to curing conditions (i.e., material **14** is in "fresh" or "green" form). Material **14** is a nicotine-containing solution (e.g., aqueous solution) that results (as a byproduct) from a process to obtain other components from green tobacco, specifically tobacco-derived protein (provided, *e.g*., as disclosed in U.S. Patent No. 9,301,544 to Mua et al.).

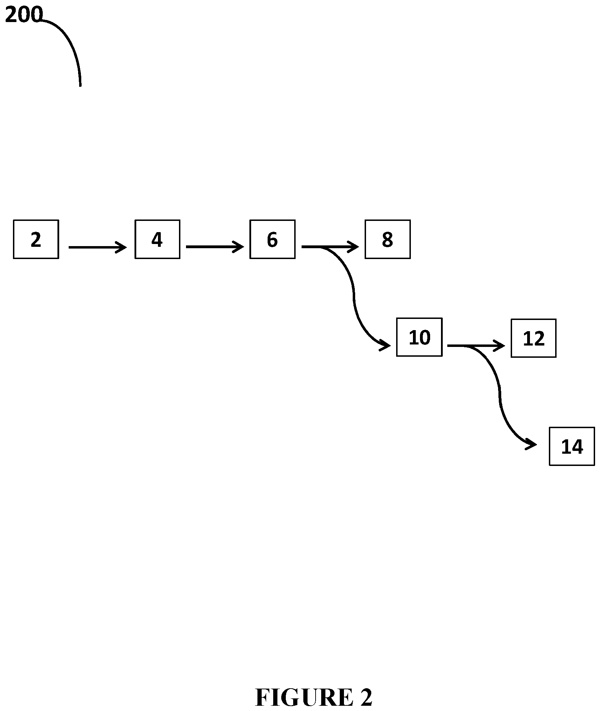

In one particular embodiment, material **14** is obtained from the process **200** shown in FIG. 2, comprising the steps of: extracting green tobacco biomass 2 with an aqueous buffer and separating the liquid component therefrom; subjecting the liquid component **4** to clarification (e.g., via decanter, disc stack, and/or filter press) to remove, e.g., starch, cells, and chlorophyll, giving clarified solution **6;** fractionating and concentrating the clarified solution to provide a protein-enriched material **8** and byproduct **10.** The byproduct of this process generally comprises small molecules that can be further processed, e.g., via evaporation to separate out a mixture of sugar, salt, and organic acids **12,** which can be further individually isolated. The byproduct of the evaporation is a distillate **14,** containing nicotine, which, as referenced above, may in some embodiments serve as the source of nicotine in the process of FIG. 1.

Advantageously, in some embodiments, to promote stability and/or for processing purposes, the nicotine in nicotine-containing material **14** is optionally processed, e.g., by filtering, evaporating, and/or condensing and is then treated (via step **A)** to convert the nicotine contained therein to the form of nicotine sulfate. Step **A** can be done, e.g., immediately following production of material **14** and/or can be conducted at a later time, *e.g.,* after material 14 has been subjected to storage for some period of time. Advantageously, this step **A** is conducted shortly after the production of material **14,** *e.g.,* within a short time period after distillate **14** is collected (e.g., within a few days, or within 24 hours).

Step **A** is generally conducted by the addition of sulfuric acid to material **14** to give nicotine sulfate-containing solution **16.** The amount of sulfuric acid added to material **14** necessarily varies, e.g., based on the amount of material **14** and the amount of nicotine contained therein. Typically, however, the amount of sulfuric acid added in step **A** is at least that amount sufficient to convert substantially all (or all) of the nicotine within material **14** to nicotine sulfate. The amount of nicotine sulfate present in the solution can vary, *e.g*., as a result of the concentration of nicotine present in material **14.** The addition of sulfuric acid generally modifies the pH of the solution as well. In some embodiments, nicotine sulfate-containing solution 16 has a pH of about 1 to about 6 or about 2 to about 6, e.g., in certain embodiments, about 2. Typically, where equipment employed for subsequent steps of the process comprise stainless steel, it may be advantageous to ensure that solution **16** has a pH at the higher end of this range (e.g., about 4 to about 6).

Advantageously, nicotine sulfate-containing solution **16** can contain fewer/different impurities than nicotine sulfate-containing solutions obtained via cured tobacco materials. For example, in some embodiments, nicotine sulfate-containing solution **16** includes fewer (including little to no) nicotine degradation products, such as nicotine oxide or myosmine. For example, in some embodiments, nicotine sulfate-containing solution **16** contains greater than 90% nicotine sulfate by dry weight, greater than 92% nicotine sulfate by dry weight, about 95% or greater nicotine sulfate by dry weight, about 96% or greater nicotine sulfate by dry weight, or about 97% or greater nicotine sulfate by dry weight. Typically, the solution **16** contains about 92% to about 98% nicotine sulfate by dry weight or about 95% to about 98% nicotine sulfate by dry weight. In some embodiments, nicotine sulfate-containing solution **16** contains about 3% or less myosmine (3-(4,5-dihydro-3H-pyrrol-2-yl)pyridine) by dry weight, and about 2% by weight or less nicotine N-oxide (1-methyl-2-(pyridine-3-yl)pyrrolidine-1-oxide). In some embodiments, nicotine sulfate-containing solution **16** contains less than 3% myosmine by dry weight or less than 2% myosmine (such as about 0.5 to about 3% or 0.5 to about 2% myosmine by dry weight, including about 1% or about 2% by dry weight). In some embodiments, nicotine sulfate-containing solution **16** contains less than 2% nicotine oxide by dry weight or less than about 1% nicotine oxide by dry weight (such as about 0.1 to about 2% nicotine oxide, about 0.5 to about 2% nicotine oxide, about 0.1 to about 1% nicotine oxide, or about 0.5 to about 1% nicotine oxide). In particular embodiments, nicotine sulfate-containing solution **16** contains less than 3% by weight myosmine and less than 2% by weight nicotine oxide, based on dry weight of the solution. In some embodiments, nicotine sulfate-containing solution **16** contains less than 2% by weight myosmine and less than 1% by weight nicotine oxide, based on dry weight. These referenced values are based on total alkaloids, and in some embodiments, water and some residual sulfuric acid may be present in nicotine sulfate-containing solution **16.**

Nicotine sulfate-containing solution **16** is further processed via concentrating step **B** to give a nicotine sulfate-containing concentrate **18.** Step **B** can be conducted by any methodology known in the art for concentrating a solution/removing liquid from a solution. Such methodologies can be conducted at various temperatures (e.g., room temperature or elevated temperature) and at various pressures (e.g., atmospheric pressure or vacuum). For example, in some embodiments, step **B** comprises vacuum evaporation. In some embodiments, step **B** comprises distillation. In some embodiments, step **B** comprises counter-current extraction.

In one particular embodiment, step **B** comprises vacuum evaporation at a slightly elevated temperature (e.g., about 40-50°C) at about -25 to about -27 in Hg (16.7 kPa to 9.90 kPa) to provide concentrate **18.** Concentrate **18** generally contains a high concentration of nicotine sulfate, e.g., at least about 20% nicotine sulfate by weight, at least about 25% nicotine sulfate by weight, at least about 30% nicotine sulfate by weight, at least about 35% nicotine sulfate by weight, or at least about 40% nicotine sulfate by weight, based on the entire weight of the concentrate, e.g., about 20% to about 45%, about 25% to about 45%, about 30% to about 45%, about 35% to about 45%, about 38% to about 42%, about 20% to about 40%, about 25% to about 40%, or about 30% to about 40% nicotine sulfate by weight, based on the entire weight of the concentrate. In one particular embodiment, concentrate **18** contains about 40% nicotine sulfate by weight, based on the entire weight of the concentrate.

Nicotine sulfate-containing concentrate **18** is then pH adjusted to a basic pH (step C) to provide basic concentrate **20,** wherein the nicotine is in free-base form (and the sulfate is, correspondingly, in the form of a sulfate salt, e.g., sodium sulfate based on the specific reagent used for pH adjustment). The reagent(s) used in step **C** to provide basic concentrate **20** can vary and a range of bases are known for pH adjustment. In certain embodiments, a sodium hydroxide solution is used to adjust pH. The pH of basic concentrate **20** is equal to or greater than that pH required for nicotine to be present as a free base (i.e. a pH of at least about 9.5). Thus, step C comprises adjusting the pH of the concentrate to a pH of about 9.5 or greater, such as about 10 or greater, about 11 or greater, about 12 or greater, or about 13 or greater. In certain embodiments, the pH range is about 12 to about 14, e.g., about 13±0.1. Where step **C** results in the formation of solids, basic concentrate **20** can be filtered (e.g., by simple/crude filtration) to remove such solids before further treatment. For example, the pH adjustment may result in the formation of sodium sulfate, which can be removed from basic concentrate **20** via crude filtration. Further, where step **C** generates heat, basic concentrate **20** can, in some embodiments, be cooled before further treatment.

It is noted that, in preferred embodiments, there is no intervening processing step between step **B** and step **C.** Various conventional methods for processing nicotine-containing solutions to purify nicotine therefrom include an organic extraction step between concentration and pH adjustment. Typically, this step is conducted to remove non-nicotine organic impurities (including, *e.g*., other alkaloids). However, due to the composition of the starting material **14,** and, correspondingly, the composition of intermediate **16** in the disclosed process (which comprises little to no non-nicotine organic impurities, as detailed herein), no such organic extraction is necessary to ultimately provide nicotine at the desired level of purity (employing the subsequent steps, as described herein below).

The pH adjusted material is subsequently subjected to liquid/liquid extraction/partitioning step **D** to give organic nicotine-containing solution **22.** In step **D,** the basic concentrate **20** is extracted with an organic solvent, which can vary. In some embodiments, the organic solvent comprises cyclohexane, but the solvent employed for extraction is understood to be not limited thereto. Various hydrocarbon solvents are effective for this stage. Advantageously, the organic solvent employed in step **D** is a solvent suitable for the extraction of nicotine therein, which is not substantially miscible with water, and which does not to any significant extent form an emulsion that is unsuitable for separation of aqueous and organic phases. In some embodiments, the organic solvent has a low boiling point so as to accommodate easy removal at a later stage of the process. Organic solvents other than cyclohexane, such as methyl tert-butyl ether (MTBE), kerosene, n-heptane, and/or n-hexane may, in some embodiments be used in step **D.** The ratio of organic solvent to basic concentrate **20** can vary; in preferred embodiments, the volume of organic solvent is less than the volume of basic concentrate 20, but the method step is not limited thereto. In one embodiment, a roughly 1:2 v/v ratio of cyclohexane to water is used.

The specific method of extraction can vary. In some embodiments, simple liquid/liquid extraction is used. In some embodiments, an industrial process such as countercurrent extraction, mixer-settler-based processes, and/or centrifugal extraction is used. Extraction is advantageously conducted at room temperature; however, in some embodiments, heat may be employed so as to conduct the extraction at an elevated temperature. The extraction can be conducted one time or multiple times to obtain the desired partitioning between aqueous and organic phases. In one embodiment, the basic concentrate **20** is mixed with organic solvent for 1 hour and phase separated to partition the nicotine into the organic phase, providing the organic nicotine-containing solution **22** (one "extraction").

The result of step **D** is an organic phase containing a majority of the nicotine originally present in the basic concentrate **20.** This organic nicotine-containing solution **22** advantageously contains at least about 85% of the nicotine or at least about 90% of the nicotine originally present in the basic concentrate **20,** wherein even higher amounts are more desirable to increase overall nicotine yield. The concentration of nicotine in the organic nicotine-containing solution **22** can vary, e.g., as a result of the amount of organic solvent employed in step **D.** In some embodiments, organic nicotine-containing solution **22** comprises about 40% to about 80% nicotine or more in cyclohexane, based on the entirety of the solution. Dry weight of nicotine in this solution (based on removal of cyclohexane) is generally about 95% or greater.

The organic nicotine-containing solution **22** is then treated via fractional distillation **E,** providing purified nicotine isolate **24.** Methods for fractional distillation are generally known to one of skill in the art, and a typical process involves heating a solution (here, organic nicotine-containing solution 22) to a temperature at which one or more components of the solution vaporizes, and the vaporized component(s) are isolated from the solution, condensed, and collected (giving a "fraction"). Various fractions are collected as the temperature of the solution is increased and, depending on the vaporization properties of the desired product (and the anticipated impurities in the solution), certain fractions will be discarded, and certain fractions will be collected and combined to give a purified solution of the desired product.

In certain embodiments, step **E** involves a 2-stage distillation. First, the organic solvent (e.g., cyclohexane) is typically removed from solution **22.** The boiling point of cyclohexane is around 80°C and, in some embodiments, this first stage is conducted over a temperature range of about 60°C to about 100°C, at atmospheric pressure. Given the composition of solution **22,** careful control over collection of the cyclohexane is not typically required, as the solution generally does not contain a high concentration of compounds that will volatilize within this range. In some embodiments, the distillate thus collected contains principally cyclohexane, although in some embodiments, it may contain trace amounts of other components (e.g., trace amounts of certain organic compounds/impurities and/or small amounts of nicotine). In some embodiments, the cyclohexane distillate can be recycled following distillation for use in additional processes **100** as the organic solvent in step **D.**

The second stage of 2-stage distillation step **E** generally involves modifying the pressure of distillation to produce a vacuum (e.g., ~3 Torr (0.400 kPa), e.g., 3±0.1 Torr (0.400±0.013 kPa) absolute). At this pressure, the boiling point of nicotine is roughly 85°C; accordingly, the temperature of the solution at this stage is raised to about 80-90°C at the noted pressure. After collection of a small amount of forerun, the nicotine is distilled from the solution, condensed, and collected until only a small portion of the original solution remains (e.g., about 5% by volume). Although not intending to be limited by theory, it is believed that this second stage of distillation removes trace amounts of various other compounds present in the organic nicotine-containing solution (e.g., other alkaloids).

The enhanced purity of the nicotine isolate **24** provided according to the disclosed process can vary. The purified nicotine isolate **24** provided according to the processes herein can be, in various embodiments, at least 99.0% nicotine, at least 99.1% nicotine, at least 99.2% nicotine, at least 99.3% nicotine, at least 99.4% nicotine, at least 99.5% nicotine, at least 99.6% nicotine, at least 99.7% nicotine, at least 99.8% nicotine, or at least 99.9% nicotine. For example, in certain embodiments, the disclosed process provides a purified nicotine isolate meeting the purity standards outlined in the USP guidelines. The USP guidelines require, *e.g*., that a nicotine extract contain not less than 99.0 percent and not more than 101.0 percent C₁₀H₁₄N₂, calculated on an anhydrous basis. In some embodiments, the disclosed process provides a purified nicotine isolate meeting EP standards. The EP standards require, *e.g*., that a nicotine extract contain less than 0.8% impurities in total, with no single impurity being present in an amount of greater than 0.4%. In some embodiments, the nicotine isolate **24** meets such criteria based on analysis by nicotine-specific high performance liquid chromatography, targeting typical impurities such as myosmine, nornicotine, nicotine oxide, cotinine, nicotyrine, anatabine, and anabasine.

In some embodiments, the purified nicotine isolate exhibits little to no color by visual inspection upon production and is substantially clear (e.g., including clear). It is noted, however, that the isolate may exhibit some yellow color over time. In some embodiments, the specific rotation as measured by a polarimeter is within the range of -130° to -152°, such as -130° to -143° or -140° to -152°. In some embodiments, the water content in the purified nicotine isolate is less than 0.5% water, as measured by Karl Fischer/toluene distillation. In some embodiments, the purified nicotine isolate comprises less than 500 ppm residual solvent as measured by headspace gas chromatography/flame ionization detection. In some embodiments, the heavy metal content of the purified nicotine isolate is less than 20 ppm (e.g., less than 20 ppm lead), as measured by a muffle furnace.

Of course, it is to be understood that various additional processes can be used within the disclosed method or in addition to the disclosed method. For example, typical separation processes can include one or more process steps such as solvent extraction (e.g., using polar solvents, organic solvents, or supercritical fluids), chromatography (e.g., preparative liquid chromatography), clarification, distillation, filtration (e.g., ultrafiltration), recrystallization, and/or solvent-solvent partitioning. Also, the parameters of various method steps can be varied. In some embodiments, it may be advantageous to conduct solvent extraction using a cold extracting liquid (e.g., water), particularly prior to a subsequent filtration step. Extractions may be conducted at elevated temperature; however, subjecting extracts resulting from such extractions directly to filtration may damage the filters, due to the heat associated with the extracts and, as such, hot/warm extracts are typically cooled prior to subsequent filtration steps. This cooling step can be avoided in some embodiments by conducting extractions at room temperature, in which case the resulting extracts can be advantageously directly treated by filtration.

As such, in some embodiments, further processing steps are incorporated within process **100** (e.g., additional heating steps, filtering steps, extraction steps, and the like). In other embodiments, process **100** consists essentially only of the steps disclosed herein (steps A-E), *i.e.,* no significant additional processing steps (e.g., additional extractions, distillations, or the like) are conducted within process **100.** The present disclosure is applicable, in some embodiments, for (in addition to laboratory, or small scale production), large/industrial scale production, where the term large scale production refers to processing large quantities of a biomass or a biomass byproduct (*e.g*., material **14** of the process of FIG. 2) on a mass production level.

It is noted that, in some embodiments, pharmaceutical-grade nicotine is provided by employing pharmaceutical quality systems and practices in manufacturing and handling the starting materials, intermediates, and products of each step of process **100.**

The process disclosed herein can employ starting material **14** derived from various forms of a plant of the *Nicotiana* species, as described for example, in US Pat. No. 9,254,001 to Byrd et al. Exemplary tobacco types from which material **14** can be obtained include, but are not limited to, Virginia (e.g., K326), burley, Indian, Kurnool, and Oriental tobaccos (including Katerini, Prelip, Komotini, Xanthi and Yambol tobaccos), Maryland, Passanda, Cubano, Jatin and Bezuki tobaccos, North Wisconsin and Galpao tobaccos, Red Russian and *Rustica* tobaccos, as well as various other rare or specialty tobaccos and various blends of any of the foregoing tobaccos. Descriptions of various types of tobaccos, growing practices and harvesting practices are set forth in Tobacco Production, Chemistry and Technology, Davis et al. (Eds.) (1999). Various representative other types of plants from the *Nicotiana* species are set forth in Goodspeed, *The Genus Nicotiana,* (Chonica Botanica) (1954); U.S. Pat. Nos. 4,660,577 to Sensabaugh, Jr. et al.; 5,387,416 to White et al., 7,025,066 to Lawson et al.; 7,798,153 to Lawrence, Jr. and 8,186,360 to Marshall et al. Exemplary *Nicotiana* species include N. tabacum, N. rustica, N. alata, N. arentsii, N. excelsior, N. forgetiana, N. glauca, N. glutinosa, N. gossei, N. kawakamii, N. knightiana, N. langsdorffi, N. otophora, N. setchelli, N. sylvestris, N. tomentosa, N. tomentosiformis, N. undulata, N. x sanderae, N. africana, N. amplexicaulis, N. benavidesii, N. bonariensis, N. debneyi, N. longiflora, N. maritina, N. megalosiphon, N. occidentalis, N. paniculata, N. plumbaginifolia, N. raimondii, N. rosulata, N. simulans, N. stocktonii, N. suaveolens, N. umbratica, N. velutina, N. wigandioides, N. acaulis, N. acuminata, N. attenuata, N. benthamiana, N. cavicola, N. clevelandii, N. cordifolia, N. corymbosa, N. fragrans, N. goodspeedii, N. linearis, N. miersii, N. nudicaulis, N. obtusifolia, N. occidentalis subsp. Hersperis, N. pauciflora, N. petunioides, N. quadrivalvis, N. repanda, N. rotundifolia, N. solanifolia, and N. spegazzinii. In some embodiments, material **14** is obtained by processing white burley tobacco, e.g., KY14 (KY14xL8) tobacco.

Descriptions of various types of tobaccos, growing practices and harvesting practices are set forth in Tobacco Production, Chemistry and Technology, Davis et al. (Eds.) (1999). Additional information on types of *Nicotiana* species suitable for use in the present invention can be found in US Pat. No. 9,107,453 to Dube et al. In some embodiments, harvested tobacco can be sprayed with a buffer or antioxidant (e.g., a sodium metabisulfite buffer) to prevent the green plants from browning prior to further treatment, to provide material **14.** Other exemplary processing techniques are described, for example, in US Pat. Nos. 7,946,295 to Brinkley et al. and 8,955,523 to Coleman, III et al. At least a portion of the plant of the *Nicotiana* species can be treated with enzymes and/or probiotics before or after harvest, as discussed in US Pat. Appl. Pub. No. 2013/0269719 to Marshall et al. and US Pat. No. 9,485,953 to Moldoveanu. *Nicotiana* species from which tobacco can be obtained for treatment as disclosed herein can be derived using genetic-modification or crossbreeding techniques (e.g., tobacco plants can be genetically engineered or crossbred to increase or decrease production of components, characteristics or attributes). See, for example, the types of genetic modifications of plants set forth in US Pat. Nos. 5,539,093 to Fitzmaurice et al.; 5,668,295 to Wahab et al.; 5,705,624 to Fitzmaurice et al.; 5,844,119 to Weigl; 6,730,832 to Dominguez et al.; 7,173,170 to Liu et al.; 7,208,659 to Colliver et al. and 7,230,160 to Benning et al.; US Patent Appl. Pub. No. 2006/0236434 to Conkling et al.; and PCT WO2008/103935 to Nielsen et al. See, also, the types of tobaccos that are set forth in US Pat. Nos. 4,660,577 to Sensabaugh, Jr. et al.; 5,387,416 to White et al.; and 6,730,832 to Dominguez et al.

The purified nicotine isolate **24** provided following the process described herein can advantageously be used in various applications. As such, the disclosure encompasses products containing purified nicotine isolate **24** and methods of providing products containing purified nicotine isolate **24.**

The purified nicotine isolate may be incorporated within a pharmaceutical product in the form of a lozenge, tablet, microtab, or other tablet-type product. Nicotine-containing pharmaceutical compositions can generally incorporate, in addition to isolate **24,** various pharmaceutically acceptable excipients. By "pharmaceutically acceptable carrier" or "pharmaceutically acceptable excipient" is intended a carrier or excipient that is conventionally used in the art to facilitate the storage, administration, and/or the healing effect of an active agent (e.g., a nicotinic compound). The carrier(s) must be pharmaceutically acceptable in the sense of being compatible with the other ingredients of the formulation and not unduly deleterious to the recipient thereof. A carrier may also reduce any undesirable side effects of the agent. See, Wang et al. (1980) J. Parent. Drug Assn. 34(6):452-462. Other exemplary pharmaceutical excipients and/or additives suitable for use in the compositions according to the invention are listed in Remington: The Science & Practice of Pharmacy, 21.sup.st ed., Lippincott Williams & Wilkins (2006); in the Physician's Desk Reference, 64.sup.th ed., Thomson PDR (2010); and in Handbook of Pharmaceutical Excipients, 6.sup.th ed., Eds. Raymond C. Rowe et al., Pharmaceutical Press (2009).

The various excipients can vary, and the selection and amount of each excipient can depend upon factors such as the ultimate form and function of product that is desired. See, for example, the types of ingredients, relative amounts and combinations of ingredients, nicotine-containing formulations and preparation processes for nicotine-containing products set forth in U.S. Pat. Nos. 5,512,306 to Carlsson et al.; 5,525,351 to Dam; 5,549,906 to Santus; 5,711,961 to Reiner et al.; 5,811,126 to Krishnamurthy; 5,939,100 to Albrechtsen et al.; 6,024,981 to Khankari et al.; 6,083,531 to Humbert-Droz et al.; 6,090,401 to Gowan, Jr. et al.; 6,110,495 to Dam; 6,426,090 to Ream et al.; 6,569,463 to Patel et al.; 6,583,160 to Smith et al.; 6,585,997 to Moro et al.; 6,893,654 to Pinney et al.; 7,025,983 to Leung et al.; and 7,163,705 Johnson et al.; US Pat. Pub. Nos. 2003/0176467 to Andersson et al.; 2003/0235617 to Martino et al.; 2004/0096501 to Vaya et al.; 2004/0191322 to Hansson; 2005/0053665 to Ek et al.; 2005/0123502 to Chan et al.; 2008/0038209 to Andersen et al.; 2008/0286341 to Andersson et al.; 2009/0023819 to Axelsson; 2009/0092573 to Andersen; and 2010/0061940 to Axelsson et al.

One particularly preferred type of a representative composition incorporating nicotine as an active ingredient, and that comprises nicotine in an orally provided form, has the form of a lozenge, tablet, microtab, or other tablet-type product. See, for example, the types of nicotine-containing lozenges, lozenge formulations, lozenge formats and configurations, lozenge characteristics and techniques for formulating or manufacturing lozenges set forth in U.S. Pat. Nos. 4,967,773 to Shaw; 5,110,605 to Acharya; 5,733,574 to Dam; 6,280,761 to Santus; 6,676,959 to Andersson et al.; 6,248,760 to Wilhelmsen; and 7,374,779 to Chen et al.; US Pat. Pub. Nos. 2001/0016593 to Wilhelmsen; 2004/0101543 to Liu et al.; 2006/0120974 to Mcneight; 2008/0020050 to Chau et al.; 2009/0081291 to Gin et al.; 2010/0004294 to Axelsson et al.; and 2013/0078307 to Holton, Jr. et al. The nicotine isolate may be sorbed onto a porous particulate carrier material, such as microcrystalline cellulose (MCC) prior to incorporation within a composition. See, for example, US Pat. Pub. No. 2004/0191322 to Hansson.

The nicotine isolate derived from methods described herein can be incorporated into an electronic smoking article. There have been proposed numerous smoking products, flavor generators, and medicinal inhalers that utilize electrical energy to vaporize or heat a volatile material, or attempt to provide the sensations of cigarette, cigar, or pipe smoking without burning tobacco to a significant degree. See, for example, the various alternative smoking articles, aerosol delivery devices and heat generating sources set forth in the background art described in U.S. Pat. No. 7,726,320 to Robinson et al., U.S. Pat. Pub. Nos. 2013/0255702 to Griffith Jr. et al., 2014/0000638 to Sebastian et al., 2014/0060554 to Collett et al., 2014/0096781 to Sears et al., 2014/0096782 to Ampolini et al., and 2015/0059780 to Davis et al.

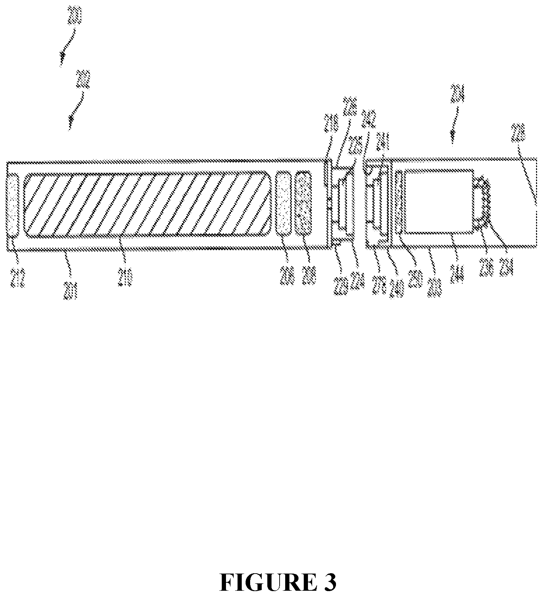

An example of an electronic smoking article 200 is shown in FIG. 3. As illustrated therein, a control body 202 can be formed of a control body shell 201 that can include a control component 206, a flow sensor 208, a battery 210, and an LED 212. A cartridge 204 can be formed of a cartridge shell 203 enclosing a reservoir housing 244 that is in fluid communication with a liquid transport element 236 adapted to wick or otherwise transport an aerosol precursor composition stored in the reservoir housing to a heater 234. An opening 228 may be present in the cartridge shell 203 to allow for egress of formed aerosol from the cartridge 204. Such components are representative of the components that may be present in a cartridge and are not intended to limit the scope of cartridge components that may be used. The cartridge 204 may be adapted to engage the control body 202 through a press-fit engagement between the control body projection 224 and the cartridge receptacle 240. Such engagement can facilitate a stable connection between the control body 202 and the cartridge 204 as well as establish an electrical connection between the battery 210 and control component 206 in the control body and the heater 234 in the cartridge. The cartridge 204 also may include one or more electronic components 250, which may include an IC, a memory component, a sensor, or the like. The electronic component 250 may be adapted to communicate with the control component 206. The various components of an electronic smoking device can be chosen from components described in the art and commercially available.

The aerosol precursor composition can comprise a nicotine isolate derived according to methods of the present disclosure. Exemplary formulations for aerosol precursor materials that may be used are described in U.S. Pat. No. 7,217,320 to Robinson et al.; U.S. Pat. Pub. Nos. 2013/0008457 to Zheng et al.; 2013/0213417 to Chong et al.; 2014/0060554 to Collett et al.; and 2014/0000638 to Sebastian et al. Other aerosol precursors that can incorporate the nicotine isolate described herein include the aerosol precursors that have been incorporated in the VUSE^{®} product by R. J. Reynolds Vapor Company, the BLU^{™} product by Imperial Tobacco, the MISTIC MENTHOL product by Mistic Ecigs, and the VYPE product by CN Creative Ltd. Also desirable are the so-called "smoke juices" for electronic cigarettes that have been available from Johnson Creek Enterprises LLC.

### EXPERIMENTAL

Aspects of the present disclosure are more fully illustrated by the following example, which is set forth to illustrate certain aspects of the present disclosure and is not to be construed as limiting thereof.

Multiple nicotine sulfate solutions (prepared by treating byproducts of protein isolation from tobacco with sulfuric acid) were combined as shown below in Table 1.

**Table 1: Starting Materials**

| Drum # | Volume (L) | pH | Nicotine mass (dry basis, g) |
|---|---|---|---|
| 1 | 195.5 | 4.93 | 196.50 |
| 2 | 203.0 | 4.91 | 199.00 |
| 3 | 199.5 | 4.21 | 476.60 |
| 4 | 195.0 | 4.31 | 528.60 |
| 5 | 198.0 | 5.04 | 442.50 |
| 6 | 199.0 | 4.96 | 435.60 |
| 7 | 146.5 | 4.83 | 266.00 |
| 8 | 146.0 | 3.08 | 259.70 |
| Total | 1482.5 | - | 2804.50 |

The combined solution was concentrated to -30% by vacuum evaporation. Specifically, the combined nicotine sulfate solution (1482.5 L) was concentrated at -27 in Hg (9.90 kPa) with a temperature setpoint of 50°C. Heat was maintained with steam. After concentration, a total of 8.9 L was obtained. Of the total, 5.2 L was through processed and 3.7 L was retained for stability studies.

The concentrate was tested at 18% nicotine sulfate by mass. The nicotine sulfate was then pH adjusted from 4.60 to 12.98 with 1.55 L of 10 M NaOH solution. After pH adjustment, the solution was extracted with 2.9 L of cyclohexane (>99.9%). The two solvents were allowed to mix for 60 mins. After mixing, the solution was left standing for 2.5 hours. Solid precipitant (sodium sulfate) settled to the bottom of the mixing vessel. The liquid portion was carefully decanted through filter paper and into a separatory funnel. The phase separation continued in the separatory funnel for 60 mins. The aqueous phase was collected from the bottom of the separatory funnel. 6.71 L of aqueous phase was collected, sampled, and discarded. The organic phase was collected separately in a total volume of 3070 mL.

The organic phase was then distilled using a 1" (25 mm) diameter spinning band fractional distillation column. Two different batch distillation methods were performed. The first method was performed to distill off cyclohexane. The second was performed under high vacuum to distill the nicotine from other organic impurities. The method parameters are outlined in Table 2 below.

**Table 2: Distillation Parameters**

| Parameter | Method 1 (cyclohexane) | Method 2 (purification) |
|---|---|---|
| Vacuum (torr) | Atmospheric | 3.00 (0.400 kPa) |
| Band speed (RPM) | 2000 | 2000 |
| Reflux (X:1)* | 5 | 15 |
| Equilibration (mins) | 30 | 150 |
| Initial Heating Rate (%) | 25 | 25 |
| Process Heating Rate (%) | 10 | 10 |
| Collection T low (°C) | 60 | 70 |
| Collection T high (°C) | 100 | 90 |
| Shutoff (°C) | 150 | 250 |

| | | |
|---|---|---|
| * Reflux (X: 1) is a ratio of how much recondensed vapor is collected as distillate (product) and how much is sent back to the distillation column (reflux), e.g., for Method 1, 5 parts reflux for every 1 part distillate was used; for Method 2, 15 parts reflux for every 1 part distillate was used. | | |

A total of 2.5 L of distilled cyclohexane was obtained from method 1, leaving 550 mL of impure nicotine to process through Method 2. A forerun fraction of 40 mL was collected and discarded. After collecting the forerun fraction, a final cut of 457 mL of purified nicotine was collected. The pure nicotine was stored in a stainless steel container under argon to preserve stability. Characterization data for the material provided by this example are provided below in Table 3, as compared against the USP and/or EP reference.

**Table 3: Characterization Data**

| **Test Parameters** | **Test Method** | **USP/EP Reference** | **Target Specification** | **Result** |
|---|---|---|---|---|
| Appearance | Visual Inspection | N/A | Colorless to Brown Liquid | Colorless Liquid |
| Identity | Infrared Absorption Spectroscopy | USP <197A> | Compares to Standard | Compares to Standard |
| Specific Rotation | Polarimeter | USP <781S> | -130° to -143° | -137.7° |
| | | EP (2.2.7) | -140° to -152° | -146.3° |
| Water | Karl Fisher/Toluene distillation | USP <921> | <0.5% | 0.05% |
| Residual Solvent | Headspace GC/FID | USP <467> | <500ppm | Pass |
| Heavy Metals | ICP-MS | USP <231> | <20ppm (Pb) | Pass |
| Purity | Non-Aqueous Titration | USP <541> | 99.0% to 101.0% | 100.0% |
| Purity | HPLC | EP (2.2.29) | Sum of impurities <0.8%, no known impurity (A-G) >0.4% | A = <0.05% |
| | | | | B = <0.05% |
| | | | | C = <0.05% |
| | | | | D = <0.05% |
| | | | | E = <0.05% |
| | | | | F = <0.05% |
| | | | | G = <0.05% |
| | | | | Unknown: None detected |

## Claims

1. A method for providing a nicotine isolate, comprising:
a) receiving a solution comprising nicotine derived from green tobacco biomass of a plant of the *Nicotiana* species which has not been cured, wherein the solution is a byproduct of a method to provide protein from tobacco;
b) converting the nicotine to nicotine sulfate, giving a nicotine sulfate-containing solution;
c) concentrating the nicotine sulfate-containing solution to give a nicotine sulfate concentrate;
d) adjusting the pH of the nicotine sulfate concentrate to a pH of about 9.5 or greater to convert the nicotine sulfate to nicotine in free base form, providing a basic concentrate;
e) extracting the basic concentrate with an organic solvent to partition the nicotine in free base form into the organic solvent, providing a nicotine-containing organic solution; and
f) distilling the nicotine-containing organic solution to afford a nicotine isolate,
wherein the nicotine isolate comprises about 90% or more nicotine by weight.

2. The method of claim 1, wherein the solution in step (a) is a distillate of a method to provide protein from tobacco.

3. The method of claim 1, wherein the converting in step b) comprises treating the solution comprising nicotine with sulfuric acid.

4. The method of claim 1, wherein the nicotine sulfate-containing solution has a pH of about 2 to about 6.

5. The method of claim 1, wherein the nicotine sulfate-containing solution comprises about 90% or more nicotine sulfate by dry weight.

6. The method of claim 1, wherein the nicotine sulfate-containing solution comprises about 3% or less myosmine by dry weight, and about 2% or less nicotine N-oxide by dry weight

7. The method of claim 1, wherein the concentrating in step c) comprises subjecting the nicotine sulfate-containing solution to vacuum evaporation or distillation, and in particular wherein the concentrating in step c) comprises vacuum evaporation at a temperature of about 40°C to about 50°C and at a pressure of about -25 in Hg to - 27 in Hg (16.7 kPa to 9.90 kPa); or
wherein the concentrating in step c) comprises subjecting the nicotine sulfate-containing solution to counter-current extraction.

8. The method of claim 1, wherein the adjusting in step d) comprises adding a sodium hydroxide solution to the nicotine sulfate concentrate.

9. The method of claim 1, wherein the adjusting in step d) comprises adjusting to a pH of about 12 or greater, and in particular wherein the adjusting in step d) comprises adjusting to a pH of about 13.

10. The method of claim 1, further comprising removing solids from the basic concentrate prior to step e), and in particular wherein the removing solids comprises filtering the solids from the basic concentrate.

11. The method of claim 1, wherein there is no intervening processing step between step d) and e).

12. The method of claim 1, wherein there is no intervening processing step between steps (c) and (d).

13. The method of claim 1, wherein there are no significant additional processing steps within the process.

14. The method of claim 1, wherein the solution referred to in step (a) is an aqueous solution.

15. The method of claim 1, wherein the organic solvent in step e) comprises cyclohexane.

16. The method of claim 1, wherein the nicotine-containing organic solution in step e) comprises at least about 40% nicotine by weight.

17. The method of claim 1, wherein the distilling of step f) comprises a first stage to remove organic solvent and a second stage to distill and collect the nicotine isolate, and in particular wherein the first stage is conducted at elevated temperature and atmospheric pressure and wherein the second stage is conducted under vacuum.

18. The method of claim 1, wherein the nicotine isolate comprises about 98% or more nicotine by weight, and in particular wherein the nicotine isolate comprises about 99% or more nicotine by weight.

## Patentansprüche

1. Verfahren zum Bereitstellen eines Nikotinisolats, umfassend:
a) Erhalten einer Lösung, die Nikotin umfasst, das aus Biomasse von grünem Tabak einer Pflanze der Art *Nicotiana* gewonnen wurde, die nicht getrocknet wurde, wobei die Lösung ein Nebenprodukt eines Verfahrens zum Bereitstellen von Protein aus Tabak ist;
b) Umwandeln des Nikotins in Nikotinsulfat, um eine nikotinsulfathaltige Lösung zu erhalten;
c) Konzentrieren der nikotinsulfathaltigen Lösung, um ein Nikotinsulfatkonzentrat zu erhalten;
d) Einstellen des pH-Werts des Nikotinsulfatkonzentrats auf einen pH-Wert von etwa 9,5 oder höher, um das Nikotinsulfat in Nikotin in freier Basenform umzuwandeln, wodurch ein basisches Konzentrat bereitgestellt wird;
e) Extrahieren des basischen Konzentrats mit einem organischen Lösungsmittel, um das Nikotin in freier Basenform in das organische Lösungsmittel abzutrennen, wodurch eine nikotinhaltige organische Lösung bereitgestellt wird; und
f) Destillieren der nikotinhaltigen organischen Lösung zu einem Nikotinisolat, wobei das Nikotinisolat etwa 90 Gew.-% oder mehr Nikotin umfasst.

2. Verfahren nach Anspruch 1, wobei die Lösung in Schritt (a) ein Destillat eines Verfahrens zum Bereitstellen von Protein aus Tabak ist.

3. Verfahren nach Anspruch 1, wobei das Umwandeln in Schritt b) das Behandeln der Nikotin umfassenden Lösung mit Schwefelsäure umfasst.

4. Verfahren nach Anspruch 1, wobei die nikotinsulfathaltige Lösung einen pH-Wert von etwa 2 bis etwa 6 aufweist.

5. Verfahren nach Anspruch 1, wobei die nikotinsulfathaltige Lösung etwa 90 % oder mehr Nikotinsulfat, bezogen auf das Trockengewicht, umfasst.

6. Verfahren nach Anspruch 1, wobei die nikotinsulfathaltige Lösung etwa 3 % oder weniger Myosmin, bezogen auf das Trockengewicht, und etwa 2 % oder weniger Nikotin-N-Oxid, bezogen auf das Trockengewicht, umfasst.

7. Verfahren nach Anspruch 1, wobei das Konzentrieren in Schritt c) das Unterziehen der nikotinsulfathaltigen Lösung einer Vakuumverdampfung oder Destillation umfasst, und insbesondere wobei das Konzentrieren in Schritt c) eine Vakuumverdampfung bei einer Temperatur von etwa 40 °C bis etwa 50 °C und bei einem Druck von etwa -25 in Hg bis -27 in Hg (16,7 kPa bis 9,90 kPa) umfasst; oder
wobei das Konzentrieren in Schritt c) das Unterziehen der nikotinsulfathaltigen Lösung einer Gegenstromextraktion umfasst.

8. Verfahren nach Anspruch 1, wobei das Einstellen in Schritt d) das Zugeben einer Natriumhydroxidlösung zu dem Nikotinsulfatkonzentrat umfasst.

9. Verfahren nach Anspruch 1, wobei das Einstellen in Schritt d) das Einstellen auf einen pH-Wert von etwa 12 oder höher umfasst, und insbesondere wobei das Einstellen in Schritt d) das Einstellen auf einen pH-Wert von etwa 13 umfasst.

10. Verfahren nach Anspruch 1, das weiter das Entfernen von Feststoffen aus dem basischen Konzentrat vor Schritt e) umfasst, und insbesondere wobei das Entfernen von Feststoffen das Filtern der Feststoffe aus dem basischen Konzentrat umfasst.

11. Verfahren nach Anspruch 1, wobei es zwischen Schritt d) und e) keinen dazwischenliegenden Verarbeitungsschritt gibt.

12. Verfahren nach Anspruch 1, wobei es zwischen den Schritten (c) und (d) keinen dazwischenliegenden Verarbeitungsschritt gibt.

13. Verfahren nach Anspruch 1, wobei es innerhalb des Prozesses keine wesentlichen zusätzlichen Verarbeitungsschritte gibt.

14. Verfahren nach Anspruch 1, wobei die in Schritt (a) genannte Lösung eine wässrige Lösung ist.

15. Verfahren nach Anspruch 1, wobei das organische Lösungsmittel in Schritt e) Cyclohexan umfasst.

16. Verfahren nach Anspruch 1, wobei die nikotinhaltige organische Lösung in Schritt e) mindestens etwa 40 Gew.-% Nikotin umfasst.

17. Verfahren nach Anspruch 1, wobei das Destillieren aus Schritt f) eine erste Stufe zum Entfernen von organischem Lösungsmittel und eine zweite Stufe zum Destillieren und Sammeln des Nikotinisolats umfasst, und insbesondere wobei die erste Stufe bei erhöhter Temperatur und Atmosphärendruck erfolgt und wobei die zweite Stufe unter Vakuum erfolgt.

18. Verfahren nach Anspruch 1, wobei das Nikotinisolat etwa 98 Gew.-% oder mehr Nikotin umfasst, und insbesondere wobei das Nikotinisolat etwa 99 Gew.-% oder mehr Nikotin umfasst.

## Revendications

1. Procédé pour fournir un isolat de nicotine, comprenant :
a) la réception d'une solution comprenant de la nicotine dérivée de la biomasse de tabac vert d'une plante de l'espèce *Nicotiana* qui n'a pas été séchée, dans lequel la solution est un sous-produit d'un procédé pour fournir des protéines à partir du tabac ;
b) la conversion de la nicotine en sulfate de nicotine, donnant une solution contenant du sulfate de nicotine ;
c) la concentration de la solution contenant du sulfate de nicotine pour donner un concentré de sulfate de nicotine ;
d) l'ajustement du pH du concentré de sulfate de nicotine à un pH d'environ 9,5 ou plus pour convertir le sulfate de nicotine en nicotine sous une forme de base libre, fournissant un concentré basique ;
e) l'extraction du concentré basique avec un solvant organique pour diviser la nicotine sous forme de base libre dans le solvant organique, fournissant une solution organique contenant de la nicotine ; et
f) la distillation de la solution organique contenant de la nicotine pour obtenir un isolat de nicotine,
dans lequel l'isolat de nicotine comprend environ 90 % ou plus de nicotine en poids.

2. Procédé selon la revendication 1, dans lequel la solution à l'étape (a) est un distillat d'un procédé pour fournir une protéine à partir du tabac.

3. Procédé selon la revendication 1, dans lequel la conversion à l'étape b) comprend le traitement de la solution comprenant de la nicotine avec de l'acide sulfurique.

4. Procédé selon la revendication 1, dans lequel la solution contenant du sulfate de nicotine présente un pH d'environ 2 à environ 6.

5. Procédé selon la revendication 1, dans lequel la solution contenant du sulfate de nicotine comprend environ 90 % ou plus de sulfate de nicotine en poids sec.

6. Procédé selon la revendication 1, dans lequel la solution contenant du sulfate de nicotine comprend environ 3 % ou moins de myosmine en poids sec et environ 2 % ou moins de N-oxyde de nicotine en poids sec

7. Procédé selon la revendication 1, dans lequel la concentration à l'étape c) comprend la soumission de la solution contenant du sulfate de nicotine à une évaporation ou une distillation sous vide et, en particulier, dans lequel la concentration à l'étape c) comprend une évaporation sous vide à une température d'environ 40 °C à environ 50 °C et à une pression d'environ -25 en Hg à -27 en Hg (16,7 kPa à 9,90 kPa) ; ou
dans lequel la concentration à l'étape c) comprend la soumission de la solution contenant du sulfate de nicotine à une extraction à contre-courant.

8. Procédé selon la revendication 1, dans lequel l'ajustement à l'étape d) comprend l'ajout d'une solution d'hydroxyde de sodium au concentré de sulfate de nicotine.

9. Procédé selon la revendication 1, dans lequel l'ajustement à l'étape d) comprend l'ajustement à un pH d'environ 12 ou plus et, en particulier, dans lequel l'ajustement à l'étape d) comprend l'ajustement à un pH d'environ 13.

10. Procédé selon la revendication 1, comprenant en outre l'élimination de solides du concentré basique avant l'étape e) et, en particulier, dans lequel l'élimination de solides comprend la filtration des solides du concentré basique.

11. Procédé selon la revendication 1, dans lequel il n'y a aucune étape de traitement intermédiaire entre les étapes d) et e).

12. Procédé selon la revendication 1, dans lequel il n'y a aucune étape de traitement intermédiaire entre les étapes (c) et (d).

13. Procédé selon la revendication 1, dans lequel il n'y a pas d'étapes de traitement supplémentaires significatives dans le processus.

14. Procédé selon la revendication 1, dans lequel la solution mentionnée à l'étape (a) est une solution aqueuse.

15. Procédé selon la revendication 1, dans lequel le solvant organique de l'étape e) comprend du cyclohexane.

16. Procédé selon la revendication 1, dans lequel la solution organique contenant de la nicotine à l'étape e) comprend au moins environ 40 % en poids de nicotine.

17. Procédé selon la revendication 1, dans lequel la distillation de l'étape f) comprend un premier stade pour éliminer le solvant organique et un second stade pour distiller et collecter l'isolat de nicotine et, en particulier, dans lequel le premier stade est mené à température et pression atmosphérique élevées et dans lequel le second stade est mené sous vide.

18. Procédé selon la revendication 1, dans lequel l'isolat de nicotine comprend environ 98 % ou plus de nicotine en poids et, en particulier, dans lequel l'isolat de nicotine comprend environ 99 % ou plus de nicotine en poids.
